(19) Europäisches Patentamt
European Patent Office
Office européen des brevets

(11) Veröffentlichungsnummer: **0 236 916 B1**

(12) # EUROPÄISCHE PATENTSCHRIFT

(45) Veröffentlichungstag der Patentschrift: **08.01.92**

(51) Int. Cl.[5]: **C07D 209/48**, C07C 251/60, A01N 43/38

(21) Anmeldenummer: **87103019.3**

(22) Anmeldetag: **04.03.87**

Die Akte enthält technische Angaben, die nach dem Eingang der Anmeldung eingereicht wurden und die nicht in dieser Patentschrift enthalten sind.

(54) **N-Aryltetrahydrophthalimidderivate und deren Vorprodukte.**

(30) Priorität: **06.03.86 DE 3607300**

(43) Veröffentlichungstag der Anmeldung:
**16.09.87 Patentblatt 87/38**

(45) Bekanntmachung des Hinweises auf die Patenterteilung:
**08.01.92 Patentblatt 92/02**

(84) Benannte Vertragsstaaten:
**AT BE CH DE ES FR GB IT LI NL SE**

(56) Entgegenhaltungen:
**EP-A- 0 006 152**
**DE-A- 2 120 087**
**GB-A- 2 150 929**
**US-B- 211 786**
**EP-A- 0 207 894**
**GB-A- 2 071 100**
**US-A- 4 260 555**

**PATENT ABSTRACTS OF JAPAN, Band 9, Nr. 317 (C-319)[2040], 12. Dezember 1985**

(73) Patentinhaber: **BASF Aktiengesellschaft**
**Carl-Bosch-Strasse 38**
**W-6700 Ludwigshafen(DE)**

(72) Erfinder: **Eicken, Karl, Dr.**
**Am Hüttenwingert 12**
**W-6706 Wachenheim(DE)**
Erfinder: **Plath, Peter, Dr.**
**Hans Balcke-Str. 13**
**W-6710 Frankenthal(DE)**
Erfinder: **Würzer, Bruno, Dr.**
**Rüdigerstr. 13**
**W-6701 Otterstadt(DE)**
Erfinder: **Meyer, Norbert, Dr.**
**Dossenheimer Weg 22**
**W-6802 Ladenburg(DE)**

EP 0 236 916 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die vorliegende Erfindung betrifft neue N-Aryltetrahydrophthalimidderivate und deren Vorprodukte der allgemeinen Formel I

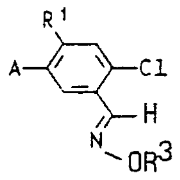

(I)

In dieser Formel I bedeuten

A   $O_2N$-, $H_2N$- oder

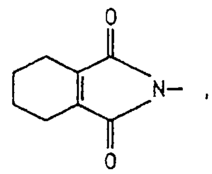

entsprechend den Verbindungen Ia, Ib und Ic,

$R^1$   Wasserstoff, Fluor oder Chlor,

$R^3$   eine $C_1$-$C_3$-Alkylgruppe, die eine $C_3$-$C_8$-Alkenyloxycarbonyl-, eine $C_3$-$C_8$-Alkinyloxycarbonylgruppe oder eine $C_2$-$C_{10}$-Alkoxycarbonylgruppe trägt, wobei die letztgenannte Gruppe im Alkoxyteil durch $C_1$-$C_4$-Alkoxy, Halogen oder Phenyl substituiert sein kann.

Außerdem betrifft die vorliegende Erfindung die Herstellung der Verbindungen I, die Verwendung der N-Aryltetrahydrophthalimidderivate Ic als Herbizide, sowie Herbizide, die die Verbindungen der Formel Ic enthalten.

Aus der GB-A-2 150 929 sind herbizid wirksame N-Aryltetrahydrophthalimide des Typs I' bekannt.

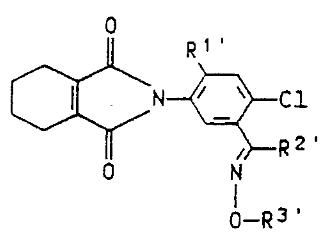

(I')

In dieser Formel bedeuten

$R^{1'}$   Wasserstoff, Halogen oder Pseudohalogen,

$R^{2'}$, $R^{3'}$   unabhängig voneinander Wasserstoff, Alkyl, Alkaryl, Araryl, Aryl, Alkoxyalkyl, Alkoxycarbonylalkyl oder Halogenalkyl.

Des weiteren werden in der JP-A 60 152 465 herbizid wirksame N-Aryltetrahydrophthalimide vom Typ der Verbindungen I' beschrieben, wobei $R^{1'}$ Wasserstoff oder Halogen, $R^{2'}$ Wasserstoff und $R^{3'}$ Wasserstoff, Halogen, Alkyl, Alkenyl oder Alkinyl bedeuten.

In der EP-A 207 894 werden herbizid wirksame N-Phenyl-3,4,5,6-tetrahydrophthalimide vom Typ I' gelehrt, wobei $R^{1'}$ Wasserstoff oder Halogen, $R^2$ insbesondere Methyl und $R^{3'}$ u.a. einen Alkoxycarbonylalkyl-, Alkenyloxycarbonylalkyl- oder Alkinyloxycarbonylalkylrest bedeuten. Dieses Dokument ist allerdings bezüglich erfinderischer Tätigkeit nur für die anmeldungsgemäßen Verbindungen 7c bis 17c, bei denen $R^3$ eine Alkoxycarbonylalkyl-, Alkenyloxycarbonylalkyl-, Alkinyloxycarbonylalkylgruppe oder eine durch Phenyl oder Alkoxy substituierte Alkoxycarbonylalkylgruppe bedeutet, relevant.

Gemäß der Lehre der US-B 211 786 sind auch Acetophenonoxime, die im Phenylteil u.a. einen gewünschtenfalls substituierten Harnstoffrest tragen können, herbizid wirksam.

Da diese bekannten Herbizide jedoch insbesondere bei niedrigen Aufwandmengen zu wünschen übrig lassen, lag der Erfindung die Aufgabe zugrunde, neue Aryltetrahydrophthalimidderivate Ic mit verbesserter herbizider Wirkung bei gleicher oder reduzierter Aufwandmenge zu entwickeln.

Demgemäß wurden die eingangs definierten neuen N-AryltetrahydrophthalimidderivateIc sowie deren Vorprodukte Ia und Ib sowie Verfahren zu deren Umsetzung gefunden. Weiterhin wurde gefunden, daß sich die Verbindungen Ic hervorragend als Herbizide eignen.

Charakteristisch für die allgemeine herbizide Wirkung der Verbindungen Ic ist die 3,4,5,6-Tetrahydrophthalimidgruppe.

Im Hinblick auf die Wirkungsverstärkung und die selektive Wirkung der Herbizide übt der Oximetherteil

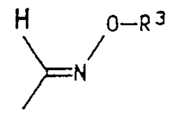

einen günstigen Einfluß aus.

Die Verbindungen Ia sind nach folgenden Methoden erhältlich:

Die Nitrobenzoloximverbindungen der Formel Ia können in Analogie zu bekannten Verfahren hergestellt werden, indem man die Nitroformylverbindung IIa entweder mit einem Hydroxylamin-Salz, z.B. Hydroxylaminhydrochlorid, in Gegenwart von Säure bindenden Mitteln zum Oxim IIa' umsetzt und dieses anschließend mit einer Verbindung $R^3$-Br umsetzt, oder indem man die Nitroformylverbindung IIa direkt mit einem O-substituierten Hydroxylamin $H_2NO-R^3$ bzw. seinem Salz gegebenenfalls in Gegenwart eines Säure bindenden Mittels umsetzt.

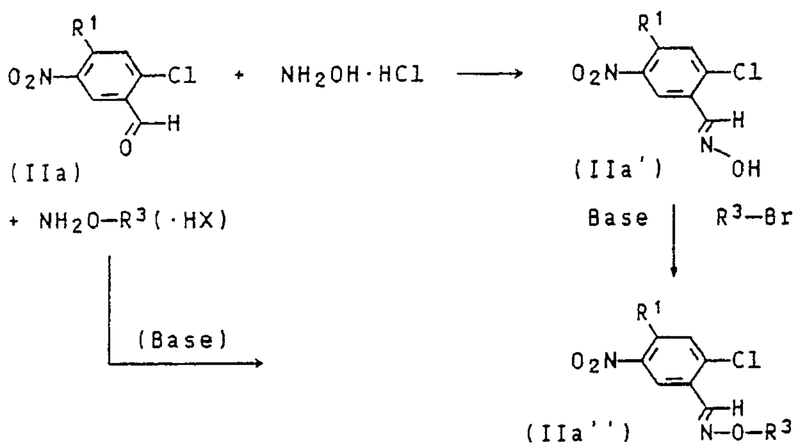


(IIa) (IIa') (IIa'')

Die Verbindungen Ib sind nach folgenden Methoden erhältlich:

Die Herstellung der Anilinoximderivate Ib erfolgt in an sich bekannter Weise durch Reduktion der Nitroverbindungen Ia. Dabei kann die Reduktion mit Salzen des zweiwertigen Zinns oder Eisens in wäßrige-saurem alkoholischem Medium wie HCl/Wasser/Ethanol durchgeführt werden, oder durch partielle katalytische Hydrierung mit Hilfe von Edelmetallkatalysatoren, z.B. Platin oder Palladium, bei Temperaturen von 0 bis 50 °C.

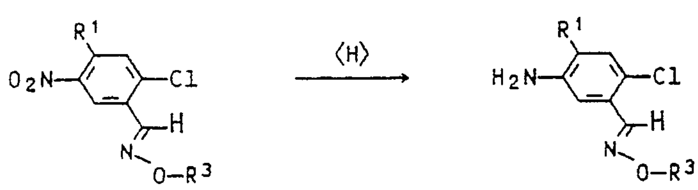


(Ia) (Ib)

Die Verbindungen Ic sind nach folgenden Methoden erhältlich:
Die N-Aryltetrahydrophthalimide Ic sind aus 3,4,5,6-Tetrahydrophthalsäureanhydrid und einem Anilinoximderivat in Abwesenheit oder Anwesenheit eines Lösungsmittels bei Temperaturen von 60 bis 200 °C erhältlich. Als Lösungsmittel eignen sich niedere Carbonsäuren, wie Eisessig oder Propionsäure oder aprotische Lösungsmittel. Beim Arbeiten in aprotischen Lösungsmitteln verwendet man zweckmäßigerweise einen Wasserabscheider um das Reaktionswasser azeotrop auszutreiben.

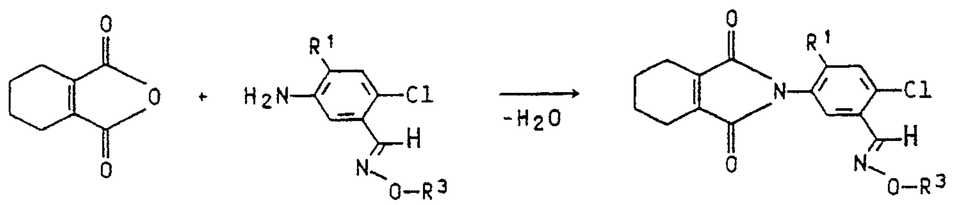

Unter den Verbindungen Ic sind diejenigen bevorzugt, in denen der Rest $R^1$ Wasserstoff oder Chlor bedeutet.

Bevorzugte Reste $R^3$ sind

- $C_1$-$C_2$-Alkyl, das eine $C_2$-$C_9$-Alkoxycarbonylgruppe trägt, besonders Methoxycarbonylmethyl, Ethoxycarbonylmethyl, Methoxycarbonyleth-1-yl, Ethoxycarbonyleth-1-yl, n-Propoxycarbonyleth-1-yl, n-Butoxycarbonylmethyl, n-Hexoxycarbonylmethyl, n-Butoxycarbonyleth-1-yl, n-Pentoxycarbonyleth-1-yl, n-Octyloxycarbonyleth-1-yl und (2-Ethyl-n-hexyloxy)-carbonyleth-1-yl,
- $C_1$-$C_2$-Alkyl, das eine $C_3$-$C_6$-Alkenyloxycarbonylgruppe trägt, besonders Allyloxycarbonylmethyl und Allyloxycarbonyleth-1-yl,
- $C_1$-$C_2$-Alkyl, das eine $C_3$-$C_6$-Alkinyloxycarbonylgruppe trägt, besonders Ethinyloxycarbonyleth-1-yl,
- $C_1$-$C_2$-Alkyl, das eine $C_2$-$C_6$-Alkoxycarbonylgruppe trägt, die im Alkoxyteil durch $C_1$-$C_4$-Alkoxy substituiert ist, besonders Methoxymethoxycarbonylmethyl, Methoxymethoxycarbonyleth-1-yl, Ethoxymethoxycarbonylethyl und Ethoxymethoxycarbonyleth-1-yl,
- $C_1$-$C_2$-Alkyl, das eine $C_2$-$C_6$-Alkoxycarbonylgruppe trägt, die im Alkoxyteil durch Phenyl substituiert ist, besonders Benzyloxycarbonylmethyl und Benzyloxycarbonyleth-1-yl.

Unter den genannten Resten $R^3$ sind Methyl, Ethyl, n-Propyl, Methoxy-und Ethoxycarbonylmethyl, Methoxy-, Ethoxy-, n-Propyloxy- und Allyloxycarbonyleth-1-yl ganz besonders bevorzugt.

Beispiele für geeignete Verbindungen sind 3,4,5,6-Tetrahydrophthalimide mit folgenden Substituenten am Imid-Stickstoff:
3-(Methoxycarbonylmethoxyiminomethyl)-4-chlorphenyl
3-(Ethoxycarbonylmethoxyiminomethyl)-4-chlorphenyl
3-(n-Propoxycarbonylmethoxyiminomethyl)-4-chlorphenyl
3-(n-Butoxycarbonylmethoxyiminomethyl)-4-chlorphenyl
3-(tert.-Butoxycarbonylmethoxyiminomethyl)-4-chlorphenyl
3-(n-Hexoxycarbonylmethoxyiminomethyl)-4-chlorphenyl
3-(Methoxycarbonyl-methylmethoxy-iminomethyl)-4-chlorphenyl
3-(Ethoxycarbonyl-methylmethoxy-iminomethyl)-4-chlorphenyl
3-(n-Propoxycarbonyl-methylmethoxy-iminomethyl)-4-chlorphenyl
3-(iso-Propoxycarbonyl-methylmethoxy-iminomethyl)-4-chlorphenyl
3-(n-Butoxycarbonyl-methylmethoxy-iminomethyl)-4-chlorphenyl
3-(Allyloxycarbonyl-methylmethoxy-iminomethyl)-4-chlorphenyl
3-(iso-Butoxycarbonyl-methylmethoxy-iminomethyl)-4-chlorphenyl
3-(tert.-Butoxycarbonyl-methylmethoxy-iminomethyl)-4-chlorphenyl
3-(n-Pentoxycarbonyl-methylmethoxy-iminomethyl)-4-chlorphenyl
3-(n-Octyloxycarbonyl-methylmethoxy-iminomethyl)-4-chlorphenyl
3-(Propargyloxycarbonyl-methylmethoxy-iminomethyl)-4-chlorphenyl
3-(Methoxycarbonyl-ethylmethoxy-iminomethyl)-4-chlorphenyl
3-(Ethoxycarbonyl-ethylmethoxy-iminomethyl)-4-chlorphenyl
3-(Methoxycarbonyl-dimethylmethoxy-iminomethyl)-4-chlorphenyl
3-(Ethoxycarbonyl-dimethylmethoxy-iminomethyl)-4-chlorphenyl
5-(Methoxyiminomethyl)-2,4-dichlorphenyl
5-(Ethoxyiminomethyl)-2,4-dichlorphenyl

5-(n-Propoxyiminomethyl)-2,4-dichlorphenyl
5-(Allyloxyiminomethyl)-2,4-dichlorphenyl
5-(Ethoxycarbonylmethoxyiminomethyl)-2,4-dichlorphenyl
5-(n-Butoxycarbonylmethoxyiminomethyl)-2,4-dichlorphenyl
5-(n-Propoxycarbonyl-methylmethoxyiminomethyl)-2,4-dichlorphenyl
5-(Ethoxycarbonylmethoxyiminomethyl)-4-chlor-2-fluorphenyl
5-(n-Butoxycarbonylmethoxyiminomethyl)-4-chlor-2-fluorphenyl
5-(n-Propoxycarbonyl-methylmethoxyiminomethyl)-4-chlor-2-fluorphenyl
3-(Methoxyiminoacetonitril)-4-chlorphenyl
3-(Ethoxyiminoacetonitril)-4-chlorphenyl
3-(Allyloxyiminoacetonitril)-4-chlorphenyl
3-(Methoxycarbonylmethoxyiminoacetonitril)-4-chlorphenyl
3-(Ethoxycarbonylmethoxyiminoacetonitril)-4-chlorphenyl
3-(n-Propoxycarbonylmethoxyiminoacetonitril)-4-chlorphenyl
3-(Methoxycarbonyl-methylmethoxyiminoacetonitril)-4-chlorphenyl
3-(Ethoxycarbonyl-methylmethoxyiminoacetonitril)-4-chlorphenyl
3-(n-Propoxycarbonyl-methylmethoxyiminoacetonitril)-4-chlorphenyl
5-(Methoxyiminoacetonitril)-2,4-dichlorphenyl
5-(Allyloxyiminoacetonitril)-2,4-dichlorphenyl
5-(Methoxycarbonylmethoxyiminoacetonitril)-2,4-dichlorphenyl
5-(Ethoxycarbonylmethoxyiminoacetonitril)-2,4-dichlorphenyl
5-(Methoxycarbonyl-methylmethoxyiminoacetonitril)-2,4-dichlorphenyl
5-(Ethoxycarbonyl-methylmethoxyiminoacetonitril)-2,4-dichlorphenyl
5-(n-Propoxycarbonyl-methylmethoxyiminoacetonitril)-2,4-dichlorphenyl

Die N-Aryltetrahydrophthalimide Ic bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich ganz nach den Verwendungszwecken; sie sollten in jedem Fall möglichst die feinste Verteilung der erfindungsgemäßen Wirkstoffe gewährleisten.

Die Verbindungen Ic eignen sich allgemein zur Herstellung von direkt versprühbaren Lösungen, Emulsionen, Pasten oder Öldispersionen aus Mineralölfraktionen von mittlerem bis hohem Siedepunkt, wie Kerosin oder Dieselöl, ferner aus Kohlenteerölen sowie aus Ölen pflanzlichen oder tierischen Ursprungs, aus aliphatischen, cyclischen und aromatischen Kohlenwasserstoffen, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali- und Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl, Octyl- oder Nonylphenol, Alkyphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Lauryl-alkoholpolyglykoletheracetat, Sorbitester, Lignin, Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel,

Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,01 bis 5,0, vorzugsweise 0,03 bis 0,5 kg/ha.

In Anbetracht des erfaßbaren Wirkungsspektrums zur Unkrautbekämpfung, der Verträglichkeit der Kulturpflanzen oder der erwünschten Beeinflussung des Wachstums derselben sowie angesichts der Vielfalt der Applikationsmethoden können die erfindungsgemäßen Verbindungen je nach Substitutionsmuster in einer großen Zahl von Kulturpflanzen eingesetzt werden.

In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |

| Botanischer Name | Deutscher Name |
|---|---|
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |

| Botanischer Name | Deutscher Name |
|---|---|
| Nicotiana tabacum (N. rustica) | Tabak |
| Olea europaea | Ölbaum |
| Oryza sativa | Reis |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die N-Aryltetrahydrophthalimidderivate der allgemeinen Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoazinderivate, Benzothiadiazinone, 2,6-Dinitroaniline, N-Phenylcarbamate, Thiolcarbamate, Halogencarbonsäuren, Triazine, Amide, andere Harnstoffe, Diphenylether, Triazinone, Uracile, Benzofuranderivate, Cyclohexan-1,3-dionderivate und andere in Betracht.

Außerdem können die neuen N-Aryltetrahydrophthalimidderivate Ic allein oder in Kombination mit anderen Herbiziden und/oder mit anderen Pflanzenschutzmitteln gemischt ausgebracht werden. Beispiele hierfür sind Mittel zur Bekämpfung von Schädlingeen oder phytopatogenen Pilzen bzw. Bakterien. Es

können Mischungen mit Mineralsalzlösungen oder auch mit nichtphytotoxischen Ölen bzw. Ölkonzentraten hergestellt werden.

## Herstellungsbeispiele

### Beispiele 1 bis 17

In eine Lösung von 100 mmol eines 2-Chlor-5-nitrobenzaldehydoximderivates und 110 mmol Bromcarbonsäureester in 140 ml trockenem Dimethylformamid wurden unter Rühren bei Raumtemperatur 200 mmol gepulvertes Kaliumcarbonat in 4 Portionen eingetragen. wonach 15 Stunden bei Raumtemperatur gerührt wurde. Das Reaktionsgemisch wurde in 600 ml kaltes Wasser eingerührt, abgesaugt und wie üblich aufgearbeitet.

Zu einer Lösung von 98 mmol des erhaltenen Produktes in 270 ml Ethanol wurden 65 ml konz. Salzsäure gegeben, 490 mmol Zinn-II-chlorid-dihydrat unter Rühren in 30 min eingetragen und anschließend 1 Stunde bei 60 °C gerührt. Nach dem Verdampfen des Ethanols im Vakuum wurde der Rückstand in eine Mischung von 155 ml 50 gew.%iger wäßriger NaOH und 400 ml Eiswasser gegeben, so daß die Temperatur +5 °C nicht überstieg, und wie üblich aufgearbeitet.

79 mmol des zuvor gebildeten Produktes und 87 mmol 3,4,5,6-Tetrahydrophthalsäureanhydrid wurden in 150 ml Eisessig 1 Stunde bei 50 °C und 7 Stunden unter Rückfluß gerührt und wie üblich nach dem Erkalten aufgearbeitet.

Die Ergebnisse sind in Tabelle 1 zusammengestellt.

Tabelle 1

Verbindungen Ia (A = $O_2N-$)

| Verbindung Nr. | $R^1$ | $R^3$ | Fp. [°C] |
|---|---|---|---|
| 1a | H | $CH_2CO_2CH_3$ | 106-107 |
| 2a | H | $CH_2CO_2C_2H_5$ | 71- 72 |
| 3a | H | $CH(CH_3)CO_2CH_3$ | 89- 90 |
| 4a | H | $CH(CH_3)CO_2C_2H_5$ | 65- 67 |
| 5a | H | $CH(CH_3)CO_2-n-C_3H_7$ | 73- 75 |
| 6a | H | $CH(CH_3)CO_2CH_2-CH=CH_2$ | 73- 74 |
| 7a | H | $CH_2-CO_2-n-C_4H_9$ | Öl |
| 8a | H | $CH_2-CO_2-n-C_6H_{13}$ | 64- 65 |
| 9a | H | $CH_2-CO_2-CH_2-CH=CH_2$ | 65- 67 |
| 10a | H | $CH(CH_3)CO_2-n-C_4H_9$ | Öl |
| 11a | H | $CH(CH_3)CO_2-n-C_5H_{11}$ | Öl |
| 12a | H | $CH(CH_3)CO_2-n-C_8H_{17}$ | Öl |
| 13a | H | $CH(CH_3)CO_2-CH_2CH(C_2H_5)n-C_4H_9$ | Öl |
| 14a | H | $CH(CH_3)CO_2-C\equiv CH$ | 110-111 |
| 15a | H | $CH(CH_3)CO_2-CH_2-$ 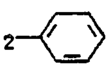 | 68- 72 |
| 16a | H | $CH(CH_3)CO_2(CH_3)OCH_3$ | 40- 45 |
| 17a | H | $CH(CH_3)CO_2(CH_2)_2OC_2H_5$ | 61- 62 |

Verbindungen Ib (A = $H_2N-$)

| Verbindung Nr. | $R^1$ | $R^3$ | Fp. [°C] |
|---|---|---|---|
| 1b | H | $CH_2CO_2CH_3$ | Öl |
| 2b | H | $CH_2CO_2C_2H_5$ | 34- 36 |
| 3b | H | $CH(CH_3)CO_2CH_3$ | Öl |
| 4b | H | $CH(CH_3)CO_2C_2H_5$ | zähe Masse |
| 5b | H | $CH(CH_3)CO_2-n-C_3H_7$ | zähe Masse |
| 6b | H | $CH(CH_3)CO_2CH_2-CH=CH_2$ | zähe Masse |
| 7b | H | $CH_2-CO_2-n-C_4H_9$ | Öl |
| 8b | H | $CH_2-CO_2-n-C_6H_{13}$ | Öl |

| Verbindung Nr. | $R^1$ | $R^3$ | Fp. [°C] |
|---|---|---|---|
| 9b | H | $CH_2-CO_2-CH_2-CH=CH_2$ | Öl |
| 10b | H | $CH(CH_3)CO_2-n-C_4H_9$ | Öl |
| 11b | H | $CH(CH_3)CO_2-n-C_5H_{11}$ | Öl |
| 12b | H | $CH(CH_3)CO_2-n-C_8H_{17}$ | Öl |
| 13b | H | $CH(CH_3)CO_2-CH_2CH(C_2H_5)-n-C_4H_9$ | Öl |
| 14b | H | $CH(CH_3)CO_2-C\equiv CH$ | Öl |
| 15b | H | $CH(CH_3)CO_2-CH_2-$ 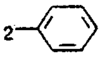 | Öl |
| 16b | H | $CH(CH_3)CO_2(CH_2)_2OCH_3$ | Öl |
| 17b | H | $CH(CH_3)CO_2(CH_2)_2OC_2H_5$ | Öl |

Verbindungen Ic

(A = 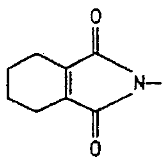 )

| Verbindung Nr. | $R^1$ | $R^3$ | Fp. [°C] |
|---|---|---|---|
| 1c | H | $CH_2CO_2CH_3$ | Öl |
| 2c | H | $CH_2CO_2C_2H_5$ | 116 - 117 |
| 3c | H | $CH(CH_3)CO_2CH_3$ | zähe Masse |
| 4c | H | $CH(CH_3)CO_2C_2H_5$ | 85 - 87 |
| 5c | H | $CH(CH_3)CO_2-n-C_3H_7$ | zähe Masse |
| 6c | H | $CH(CH_3)CO_2CH_2-CH=CH_2$ | zähe Masse |
| 7c | H | $CH_2-CO_2-n-C_4H_9$ | 91 - 92 |
| 8c | H | $CH_2-CO_2-n-C_6H_{13}$ | 64 - 66 |
| 9c | H | $CH_2-CO_2-CH_2-CH=CH_2$ | 83 - 86 |
| 10c | H | $CH(CH_3)CO_2-n-C_4H_9$ | Öl |
| 11c | H | $CH(CH_3)CO_2-n-C_5H_{11}$ | Öl |
| 12c | H | $CH(CH_3)CO_2-n-C_8H_{17}$ | Öl |
| 13c | H | $CH(CH_3)CO_2-CH_2CH(C_2H_5)-n-C_4H_9$ | Öl |
| 14c | H | $CH(CH_3)CO_2-C\equiv CH$ | Öl |
| 15c | H | $CH(CH_3)CO_2-CH_2-$ 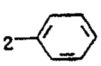 | Öl |
| 16c | H | $CH(CH_3)CO_2(CH_2)_2OCH_3$ | Öl |
| 17c | H | $CH(CH_3)CO_2(CH_2)_2OC_2H_5$ | Öl |

## Anwendungsbeispiele

Die herbizide Wirkung der N-Aryltetrahydrophthalimidderivate der allgemeinen Formel Ic auf das Wachstum der Testpflanzen wurde durch folgende Gewächshausversuche gezeigt.

Als Kulturgefäße dienten Plastikblumentöpfe mit 300 $cm^3$ Inhalt und lehmigem Sand mit etwa 3,0 % Humus als Substrat. Die Samen der Testpflanzen wurden nach Arten getrennt flach eingesät.

Bei Vorauflaufbehandlung wurden die Wirkstoffe unmittelbar danach auf die Erdoberfläche aufgebracht. Sie wurden hierbei in Wasser als Verteilungsmittel suspendiert oder emulgiert und mittels fein verteilender Düsen gespritzt. Die Aufwandmenge betrug 3,0 kg Wirkstoff/ha. Nach dem Aufbringen der Mittel wurden die Gefäße leicht beregnet, um Keimung und Wachstum in Gang zu bringen. Danach deckte man die Gefäße mit durchsichtigen Plastikhauben ab, bis die Pflanzen angewachsen sind. Diese Abdeckung bewirkte ein gleichmäßiges Keimen der Testpflanzen, sofern dies nicht durch die Wirkstoffe beeinträchtigt wurde.

Zum Zwecke der Nachauflaufbehandlung zog man die Testpflanzen je nach Wuchsform erst bis zu einer Wuchshöhe von 3 bis 15 cm an und behandelt sie danach. Es wurden direkt gesäte und in den gleichen Gefäßen aufgewachsene Pflanzen ausgewählt, oder sie wurden erst als Keimpflanzen getrennt angezogen. Die Aufwandmengen für die Nachauflaufbehandlung variierten je nach Wirkstoff, sie betrugen 0,03 bis 0,06 kg Wirkstoff/ha. Eine Abdeckung unterblieb bei der Nachauflaufbehandlung.

Die Versuchsgefäße wurden im Gewächshaus aufgestellt, wobei für warmeliebende Arten wärmere Bereiche (20 bis 36 °C) und für solche gemäßigter Klimate 10 bis 20 °C bevorzugt wurden. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Die in den Gewächshausversuchen verwendeten Pflanzen setzten sich aus folgenden Arten zusammen:

| Lateinischer Name | Deutscher Name |
|---|---|
| Abutilon theophrasti | Chinesischer Hanf |
| Amaranthus retroflexus | Zurückgekrümmter Fuchsschwanz |
| Arachis hypogaea | Erdnuß |
| Avena sativa | Hafer |
| Chenopodium album | Weißer Gänsefuß |
| Chrysanthemum coronarium | Kronenwucherblume |
| Echinochloa crus-galli | Hühnerhirse |
| Galium aparine | Klettenlabkraut |
| Ipomoea spp. | Prunkwindearten |
| Lamium amplexicaule | Stengelumfassende Taubnessel |
| Lolium multiflorum | Ital. Raygras |
| Mercurialis annua | Einjähriges Bingelkraut |
| Polygonum aviculare | Vogelknöterich |
| Solanum nigrum | Schwarzer Nachtschatten |
| Stellaria media | Vogelsternmiere |
| Triticum aestivum | Weizen |

Verbindung 9c eignete sich mit 0,03 kg Wirkstoff/ha zur Bekämpfung eines breiten Spektrums unerwünschter Pflanzen, wobei Weizen als Kulturpflanze kaum geschädigt wird. Es handelt sich um einen selektiv herbiziden Wirkstoff.

Im Vergleich mit dem bekannten Wirkstoff A aus GB-A-2 150 929

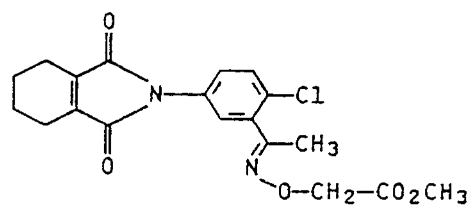

zeigte Verbindung 1c gegen eine Reihe unerwünschter Pflanzen deutlich stärkere herbizide Eigenschaften.

## Patentansprüche

**Patenansprüche für folgende Vertragsstaaten : BE, CH, DE, FR, GB, IT, LI, NL, SE**

1. N-Aryltetrahydrophthalimidderivate und deren Vorprodukte der allgemeinen Formel I

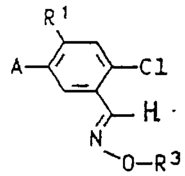

(I)

in der die Substituenten folgende Bedeutung haben:

A $O_2N$-, $H_2N$- oder

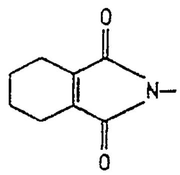

entsprechend den Verbindungen Ia, Ib und Ic,

$R^1$ Wasserstoff, Fluor oder Chlor,

$R^3$ eine $C_1$-$C_3$-Alkylgruppe, die eine $C_3$-$C_8$-Alkenyloxycarbonyl-, eine $C_3$-$C_8$-Alkinyloxycarbonylgruppe oder eine $C_2$-$C_{10}$-Alkoxycarbonylgruppe trägt, wobei die letztgenannte Gruppe im Alkoxyteil durch $C_1$-$C_4$-Alkoxy, Halogen oder Phenyl substituiert sein kann.

**2.** Verfahren zur Herstellung der Nitrobenzoloximderivate Ia gemäß Anspruch 1,

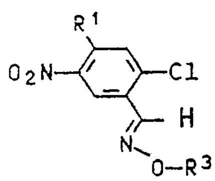

(Ia)

dadurch gekennzeichnet, daß man die entsprechende Nitroformylverbindung

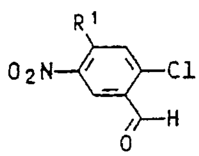

mit einem Hydroxylamin-Salz und dann mit $R^3$-Halogen oder mit O-substituiertem Hydroxylamin $H_2N$-O-$R^3$ direkt umsetzt.

**3.** Verfahren zur Herstellung von Anilinoximderivaten der Formel Ib gemäß Anspruch 1

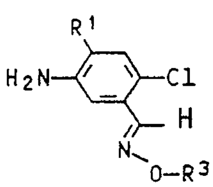

(Ib) ,

dadurch gekennzeichnet, daß man ein Nitrobenzoloximderivat der Formel Ia gemäß Anspruch I in an

sich bekannter Weise zum Anilinoximderivat der Formel Ib gemäß Anspruch 1 reduziert.

4. Verfahren zur Herstellung der N-Aryltetrahydrophthalimidderivate der Formel Ic gemäß Anspruch 1

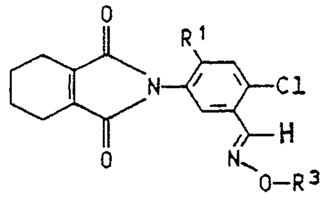

(Ic) ,

dadurch gekennzeichnet, daß man 3,4,5,6-Tetrahydrophthalsäureanhydrid in an sich bekannter Weise mit einem Anilinoximderivat Ib gemäß Anspruch 1 umsetzt.

5. Herbizides Mittel, enthaltend neben Zusatzstoffen ein N-Aryltetrahydrophthalimidderivat der Formel Ic gemäß Anspruch 1.

6. Verfahren zur Bekämpfung unerwünschten Pflanzenwuchses, dadurch gekennzeichnet, daß man eine herbizid wirksame Menge einer Verbindung Ic gemäß Anspruch 1 verwendet.

**Patentansprüche für folgende Vertragsstaaten : AT, ES**

1. Verfahren zur Herstellung der N-Aryltetrahydrophthalimidderivate der Formel Ic

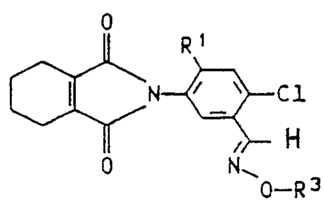

(Ic)

in der die Substituenten folgende Bedeutung haben:

$R^1$ Wasserstoff, Fluor oder Chlor,

$R^3$ eine $C_1$-$C_3$-Alkylgruppe, die eine $C_3$-$C_8$-Alkenyloxycarbonyl-, eine $C_3$-$C_8$-Alkinyloxycarbonylgruppe oder eine $C_2$-$C_{10}$-Alkoxycarbonylgruppe trägt, wobei die letztgenannte Gruppe im Alkoxyteil durch $C_1$-$C_4$-Alkoxy, Halogen oder Phenyl substituiert sein kann,

dadurch gekennzeichnet, daß man 3,4,5,6-Tetrahydrophthalsäureanhydrid mit einem Anilinoximderivat Ib

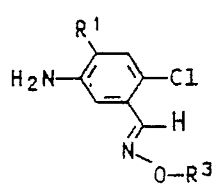

(Ib)

umsetzt.

2. Verfahren zur Herstellung von Anilinoximderivaten der Formel Ib gemäß Anspruch 1, dadurch gekennzeichnet, daß man ein Nitrobenzoloximderivat der Formel Ia

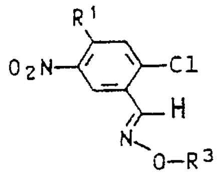

(Ia) ,

wobei die Substituenten folgende Bedeutung haben:

$R^1$ Wasserstoff, Fluor oder Chlor,
$R^3$ eine $C_1$-$C_3$-Alkylgruppe, die eine $C_3$-$C_8$-Alkenyloxycarbonyl-, eine $C_3$-$C_8$-Alkinyloxycarbonylgruppe oder eine $C_2$-$C_{10}$-Alkoxycarbonylgruppe trägt, wobei die letztgenannte Gruppe im Alkoxyteil durch $C_1$-$C_4$-Alkoxy, Halogen oder Phenyl substituiert sein kann,

zum Anilinoximderivat der Formel Ib reduziert.

3. Verfahren zur Herstellung der Nitrobenzoloximderivate der Formel Ia gemäß Anspruch 2, dadurch gekennzeichnet, daß man die entsprechende Nitroformylverbindung

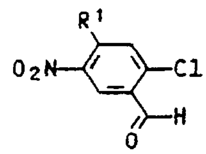

in der $R^1$ Wasserstoff, Fluor oder Chlor bedeutet, mit einem Hydroxylamin-Salz und dann mit $R^3$-Halogen oder mit O-substituiertem Hydroxylamin $H_2N$-O-$R^3$ direkt umsetzt.

4. Herbizide Mittel, enthaltend neben Zusatzstoffen N-Aryltetrahydrophthalimidderivateder Formel Ic gemäß Anspruch 1.

5. Herbizide Mittel, enthaltend neben Zusatzstoffen N-Aryltetrahydrophthalimidderivateder Formel Ic gemäß Anspruch 1, wobei die Substituenten folgende Bedeutung haben:

$R^1$ Wasserstoff oder Chlor,
$R^3$ eine $C_1$-$C_2$-Alkylgruppe, die eine $C_3$-$C_8$-Alkenyloxycarbonyl-, eine $C_3$-$C_8$-Alkinyloxycarbonylgruppe oder eine $C_2$-$C_9$-Alkoxycarbonylgruppe trägt, wobei die letztgenannte Gruppe im Alkoxyteil durch $C_1$-$C_4$-Alkoxy oder Phenyl substituiert sein kann.

6. Verfahren zur Bekämpfung unerwünschten Pflanzenwuchses, dadurch gekennzeichnet, daß man eine herbizid wirksame Menge einer Verbindung Ic verwendet, wie sie gemäß Anspruch 1 erhalten werden kann.

**Claims**
**Claims for the following Contracting States : BE, CH, DE, FR, GB, IT, LI, NL, SE**

1. An N-aryltetrahydrophthalimide derivative or a precursor therefor of the general formula I

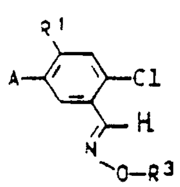

(I)

where the substituents have the following meanings:
A is $O_2N$-, $H_2N$- or

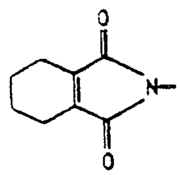 ,

corresponding to compound Ia, Ib or Ic,

$R^1$ is hydrogen, fluorine or chlorine,

$R^3$ is $C_1$-$C_3$-alkyl which carries a $C_3$-$C_8$-alkenyloxycarbonyl, a $C_3$-$C_8$-alkynyloxycarbonyl or a $C_2$-$C_{10}$-alkoxycarbonyl group, the last of which may be substituted in the alkoxy moiety by $C_1$-$C_4$-alkoxy, halogen or phenyl.

**2.** A process for preparing a nitrobenzeneoxime derivative Ia as claimed in claim 1,

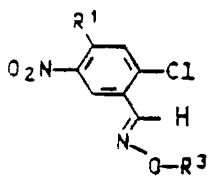 (Ia)

which comprises reacting the corresponding nitroformyl compound

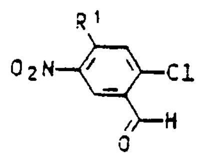

directly with a hydroxylamine salt and then with $R^3$-halogen or with O-substituted hydroxylamine $H_2N$-$O$-$R^3$.

**3.** A process for preparing an anilineoxime derivative of the formula Ib as claimed in claim 1

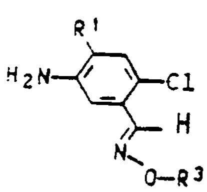 (Ib),

which comprises reducing a nitrobenzeneoxime derivative of the formula Ia as claimed in claim 1 to the anilineoxime derivative of the formula Ib as claimed in claim 1 in a conventional manner.

**4.** A process of preparing an N-aryltetrahydrophthalimide derivative of the formula Ic as claimed in claim 1

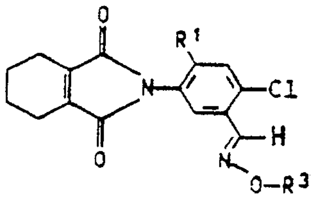

(Ic),

which comprises reacting 3,4,5,6-tetrahydrophthalic anhydride with an anilineoxime derivative Ib as claimed in claim 1 in a conventional manner.

5. A herbicidal composition containing, in addition to additives, an N-aryltetrahydrophthalimide derivative of the formula Ic as claimed in claim 1.

6. A process for controlling undesirable plant growth, which comprises using a herbicidally effective amount of a compound Ic as claimed in claim 1.

**Claims for the following Contracting States : AT, ES**

1. A process for preparing an N-aryltetrahydrophthalimide derivative of the formula Ic

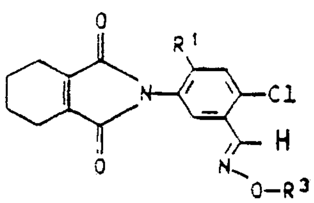

(Ic)

where the substituents have the following meanings:

$R^1$ is hydrogen, fluorine or chlorine,
$R^3$ is $C_1$-$C_3$-alkyl which carries a $C_3$-$C_8$-alkenyloxycarbonyl, a $C_3$-$C_8$-alkynyloxycarbonyl or a $C_2$-$C_{10}$-alkoxycarbonyl group, the last of which may be substituted in the alkoxy moiety by $C_1$-$C_4$-alkoxy, halogen or phenyl, which comprises reacting 3,4,5,6-tetrahydrophthalic anhydride with an anilineoxime derivative Ib

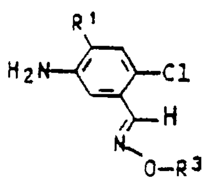

(Ib)

2. A process for preparing an anilineoxime derivative of the formula Ib as set forth in claim 1, which comprises reducing a nitrobenzeneoxime derivative of the formula Ia

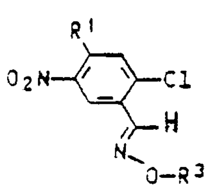

(Ia)

where the substituents have the following meanings:

- $R^1$ is hydrogen, fluorine or chlorine,
- $R^3$ is $C_1$-$C_3$-alkyl which carries a $C_3$-$C_8$-alkenylorycarbonyl, a $C_3$-$C_8$-alkynyloxycarbonyl or a $C_2$-$C_{10}$-alkoxycarbonyl group, the last of which may be substituted in the alkoxy moiety by $C_1$-$C_4$-alkoxy, halogen or phenyl,

to the anilineoxime derivative of the formula Ib.

3. A process for preparing a nitrobenzeneoxime derivative of the formula Ia as set forth in claim 2, which comprises reacting the corresponding nitroformyl compound

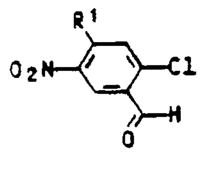

where $R^1$ is hydrogen, fluorine or chlorine, directly with a hydroxylamine salt and then with $R^3$-halogen or with O-substituted hydroxylamine $H_2N$-O-$R^3$.

4. A herbicidal composition containing, in addition to additives, an N-aryltetrahydrophthalimide derivative of the formula Ic as claimed in claim 1.

5. A herbicidal composition containing, in addition to additives, an N-aryltetrahydrophthalimide derivative of the formula Ic as claimed in claim 1 where the substituents have the following meanings:

- $R^1$ is hydrogen or chlorine,
- $R^3$ is $C_1$-$C_2$-alkyl which carries a $C_3$-$C_8$-alkenyloxycarbonyl, a $C_3$-$C_8$-alkynyloxycarbonyl or a $C_2$-$C_9$-alkoxycarbonyl group, of which the last group may be substituted in the alkoxy moiety by $C_1$-$C_4$-alkoxy or phenyl.

6. A process for controlling undesirable plant growth, which comprises using a herbicidally effective amount of a compound Ic as can be obtained as claimed in claim 1.

**Revendications**

**Revendications pour les Etats contractants suivants : BE, CH DE, FR, GB, IT, LI, NL, SE**

1. Dérivés de N-aryltetrahydrophtalimide et leurs produits intermédiaires de la formule générale I

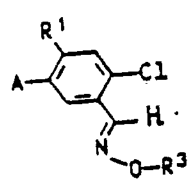

dans laquelle les substituants ont la signification suivante

A, $O_2N$-, $H_2N$- ou

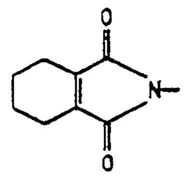

correspondant aux composés Ia, Ib et Ic.

$R^1$, hydrogène, fluor ou chlore

$R^3$, un groupe alkyle en $C_1$-$C_3$, qui porte un groupe (alcényle en $C_3$-$C_8$)-oxycarbonyle, un groupe (alcinyle en $C_3$-$C_8$)-oxycarbonyle ou un groupe (alcoxy en $C_2$-$C_{10}$)-carbonyle, le dernier groupe indiqué pouvant être substitué dans la partie alcoxy par alcoxy en $C_1$-$C_4$, halogène ou phenyle.

**2.** Procédé de préparation des dérivés de nitrobenzoloxime $I_a$ selon la revendication 1,

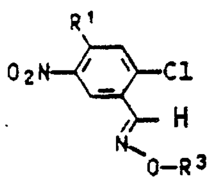

caractérisé par le fait qu'on fait réagir directement le composé nitroformyle correspondant

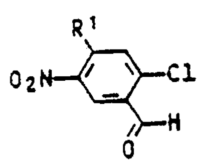

avec un sel d'hydroxylamine puis avec $R^3$ halogène ou avec hydroxylamine substituée en O, $H_2N$-O-$R^3$.

**3.** Procédé de préparation de dérivés d'anilinoxime de formule $I_b$ selon la revendication,

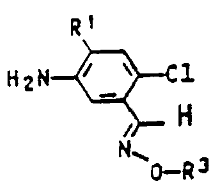

caractérisé par le fait que l'on réduit un dérivé de nitrobenzoloxime de la formule $I_a$ selon la revendication 1, de manière connue, en dérivé d'anilinoxime de formule $I_b$ selon la revendication 1.

**4.** Procédé de préparation des dérivés de N-aryltetrahydrophtalimide de la formule $I_c$ selon la revendication 1

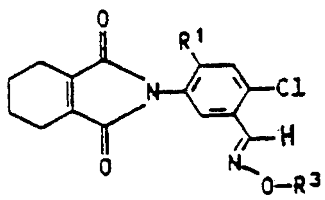

caractérisé par le fait que l'on fait réagir de l'anhydride d'acide 3, 4, 5, 6-tetrahydrophtalique, de manière connue, avec un dérivé d'anilinoxime $I_b$ selon la revendication 1.

5. Agent herbicide contenant outre des additifs un dérivé de N-aryltetrahydrophtalimide de formule $I_c$ selon la revendication 1.

6. Procédé pour lutter contre la croissance des plantes indésirables, caractérisé par le fait que l'on utilise une quantité efficace au point de vue herbicide, d'un composé $I_c$ selon la revendication 1.

**Revendications pour les Etats contractants suivants : AT, ES**

1. Procédé de préparation des dérivés de N-aryltetrahydrophtalimide de la formule $I_c$

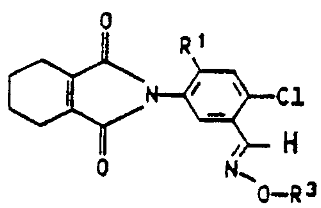

dans laquelle les substituants ont la signification suivante

- $R^1$, hydrogène, fluor ou chlore
- $R^3$, un groupe alkyle en $C_1$-$C_3$, qui porte un groupe (alcényle en $C_3$-$C_8$)-oxycarbonyle, un groupe (alcinyle en $C_3$-$C_8$)-oxycarbonyle ou un groupe (alcoxy en $C_2$-$C_{10}$)-carbonyle, le dernier groupe indiqué pouvant être substitué dans la partie alcoxy par alcoxy en $C_1$-$C_4$, halogène ou phenyle.

caractérisé par le fait que l'on fait réagir de l'anhydride d'acide 3, 4, 5, 6-tetrahydrophtalique, de manière connue, avec un dérivé d'anilinoxime $I_b$ selon la revendication 1.

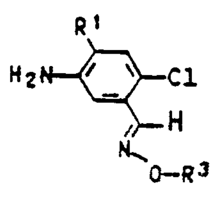

2. Procédé de préparation de dérivés d'anilinonoxime de la formule $I_b$ selon la revendication 1, caractérisé par le fait que l'on réduit en anilinoxime de formule $I_b$ un dérivé de nitrobenzoloxime de la formule $I_a$

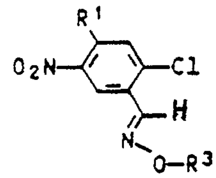

où les substituants ont la signification suivante

- $R^1$, hydrogène, fluor ou chlore
- $R^3$, un groupe alkyle en $C_1$-$C_3$, qui porte un groupe (alcényle en $C_3$-$C_8$)-oxycarbonyle, un groupe (alcinyle en $C_3$-$C_8$)-oxycarbonyle ou un groupe (alcoxy en $C_2$-$C_{10}$)-carbonyle, le dernier groupe indiqué pouvant être substitué dans la partie alcoxy par alcoxy en $C_1$-$C_4$, halogène ou phenyle.

**3.** Procédé de préparation des dérivés de nitrobenzoloxime de la formule $I_a$ selon la revendication 2 caractérisé par le fait que l'on fait réagir directement le composé nitroformyle correspondant

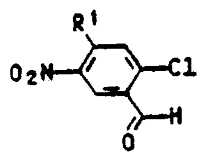

dans lequel $R^1$ représente hydrogène, fluor ou chlore avec un sel d'hydroxylamine puis avec $R^3$-halogène ou avec hydroxylamine substituée en O, $H_2N$-O-$R^3$.

**4.** Agent herbicide contenant outre des additifs un dérivé de N-aryltetrahydrophtalimide de formule $I_c$ selon la revendication 1.

**5.** Agent herbicide contenant outre des additifs un dérivé de N-aryltetrahydrophtalimide de formule $I_c$ selon la revendication 1, les substituants ayant la signification suivante

- $R^1$, hydrogène, fluor ou chlore
- $R^3$, un groupe alkyle en $C_1$-$C_3$, qui porte un groupe (alcényle en $C_3$-$C_8$)-oxycarbonyle, un groupe (alcinyle en $C_3$-$C_8$)-oxycarbonyle ou un groupe (alcoxy en $C_2$-$C_{10}$)-carbonyle, le dernier groupe indiqué pouvant être substitué dans la partie alcoxy par alcoxy en $C_1$-$C_4$, halogène ou phenyle.

**6.** Procédé pour lutter contre la croissance des plantes indésirables, caractérisé par le fait que l'on utilise une quantité efficace, du point de vue herbicide, d'un composé $I_c$, tel qu'il peut être obtenu selon la revendication 1.